# EUROPEAN PATENT SPECIFICATION

(11) **EP 3 993 777 B1**
(45) Date of publication and mention of the grant of the patent: **12.06.2024**
(21) Application number: 20750358.2
(22) Date of filing: 03.07.2020
(51) Int. Cl.: A61K 9/16, A61K 9/20, A61K 47/26, A61K 47/38, A61K 31/506, A61P 9/12

(54) **COMPRESSED MACITENTAN COMPOSITIONS, METHODS AND USES THEREOF**
GEPRESSTE MACITENTANZUSAMMENSETZUNGEN, VERFAHREN UND VERWENDUNGEN DAVON
COMPOSITIONS DE MACITENTAN COMPRIMÉ, PROCÉDÉS ET UTILISATIONS ASSOCIÉS

(30) Priority: 05.07.2019 PT 2019115632; 08.07.2019 EP 19185065
(43) Date of publication of application: 11.05.2022
(73) Proprietor: Tecnimede, Sociedade Técnico-Medicinal, SA, 2710-089 Sintra (PT)
(72) Inventor: SILVA SERRA, João Pedro, 2710-089 Sintra (PT); FERREIRA PEREIRA, Ricardo José Camilo, 2710-089 Sintra (PT)
(74) Representative: Patentree
(86) International application number: PCT/IB2020/056304
(87) International publication number: WO 2021/005478

(56) References cited:
- WO-A1-2010/054845
- WO-A1-2014/173805
- WO-A1-2018/153925
- CN-A- 107 913 256
- CN-A- 109 260 163
- US-B2- 8 367 685
- Anonymous: "Handbook of Pharmaceutical Excipients" In: "Lactose, Maltitol, Mannitol, Xylitol", 1 January 2006 (2006-01-01), Pharmaceutical Press and the Americal Pharmacists Association, Frome, Sommerset, Great Britain, XP055657295, page 385-388,438-439,449-453,824-827, the whole document

## Description

### Technical field

The present invention relates to compressed compositions comprising a therapeutically effective amount of macitentan, a sugar alcohol diluent and a surfactant. It also relates to the process of obtaining said compressed compositions, oral pharmaceutical compositions comprising them, and their use in the treatment of pulmonary arterial hypertension.

### Background

Macitentan is the international non-proprietary name (INN) of the chemical compound N-[5-(4-Bromophenyl)-6-[2-[(5-bromo-2-pyrimidinyl)oxy]ethoxy]-4-pyrimidinyl]-N'propylsulfamide, also known by the code name ACT-064992 . Its molecular formula is C₁₉H₂₀Br₂N₆O₄S and its molecular weight is 588.27 g.mol⁻¹. It has the following structural formula:

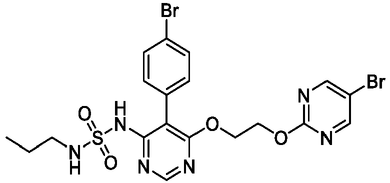

Macitentan is an endothelin receptor antagonist (ERA) currently approved and sold in Europe (EU and other countries), USA and Japan, under the trademark Opsumit, for the treatment of pulmonary arterial hypertension. Macitentan is a dual endothelin (ET) receptor antagonist that selectively inhibits the binding of ET-1 to ET_{A} and ET_{B} receptors. Macitentan inhibits the effects mediated by these receptors.

Macitentan is insoluble in aqueous solutions at room temperature at pH 1.2, 4, 6.8, 7, 9, and is only slightly soluble in organic solvents methanol and ethanol. It is a Biopharmaceutics Classification System (BCS) Class II compound, characterized by a high permeability and low solubility. Its solvation rate limits the bioavailabity exhibited *in vivo.*

MCT-IMA is the most prevalent impurity of macitentan formed either by oxidation or hydrolysis. It is also the major metabolite excreted in faeces. (Yerra, N. V. et al. Rapid Commun Mass Spectrum 2018, 15, 32, (13), 1075-1084; Thummar, M. et al. Chromatographia 2018, 81, 3, 525-531) (Bruderer, S. et al. Xenobiotica, 2012, 1-10).

Document WO 2002053557 A1 discloses macitentan and its preparation method.

Document WO 2007031933 and US8367685B2 discloses solid oral pharmaceutical compositions of macitentan. The results hint that, of all the possibilities tested, the compositions evidencing better behaviour were the ones comprising a high dosage of lactose or lactose monohydrate as filler.

Document IN 201641023754 discloses macitentan compositions comprising lactose as a filler.

Document WO 2014173805 discloses macitentan compositions comprising a specific polymorphic form. In particular, document WO 2014173805 discloses the preparation of a pharmaceutical composition comprising crystalline macitentan free base.

Document WO 2018153925 discloses macitentan compositions which does not contain surfactants.

Document IN 201641002749 discloses macitentan compositions obtained by extrusion method.

Document CN107913256 relates to macitentan-disintegrating tablet contained the following component ratio: macitentan-5-10%, a disintegrant 5-20%, the adhesive 0.5-20%, lubricant 0.5-5%, flavouring agent 2-5%, filler 65-75 mg.

Document CN109260163 A relates to a stable macitentan tablet composition, to provide a qualitative clinical medicine for the treatment of pulmonary hypertension, whereby the tablet composition contains macitentan, Tween 80, calcium phosphate, microcrystalline cellulose, anhydrous lactose, microcristalline silica gel powder, croscarmellose sodium, and magnesium stearate.

Document WO 2010054845 A1 relates to a method for the preparation of calcium carbonate tablets, which method results in tablets that have improved storage stability with respect to dissolution.

These facts are disclosed in order to illustrate the technical problem addressed by the present disclosure.

### General Description

In view of the drawbacks to the prior art, we hereby disclose solid oral pharmaceutical compositions comprising a therapeutically effective amount of macitentan that simultaneously display improved stability properties and adequate dissolution profiles.

This disclosure relates to compressed compositions comprising a therapeutically effective amount of macitentan, 15% to 85% (wt_{sugar alcohol diluent}/wtₜₒₜₐₗ) of a sugar alcohol diluent selected from a list consisting of: maltitol, xylitol, mannitol; and 0.1% to 15% (wt_{surfactant}/wtₜₒₜₐₗ) of a surfactant selected from a list consisting of: sodium dodecyl sulfate (SDS), polysorbate 80 (Tween 80). It also relates to the process of obtaining said compressed compositions, oral pharmaceutical compositions comprising them, and their use in the treatment of pulmonary arterial hypertension.

The present disclosure also relates to solid oral pharmaceutical compositions comprising a therapeutically effective amount of macitentan that simultaneously display improved stability properties and adequate dissolution profiles.

In an embodiment, the surfactant refers to polysorbate 80 (polyoxyethylene (20) sorbitan monooleate) orSDS (sodium dodecyl sulfate). Preferably, the surfactant will be selected from polysorbate 80 (polyoxyethylene (20) sorbitan monooleate) or SDS (sodium dodecyl sulfate).

In an embodiment for better results, said surfactant is sodium dodecyl sulfate, or polysorbate 80.

In an embodiment, the sugar alcohol diluent refers to xylitol, mannitol, or maltitol.

In a preferred embodiment, the surfactant is sodium dodecyl sulfate (SDS).

In a preferred embodiment, the surfactant is polysorbate 80 (Tween 80).

In one embodiment, the compressed composition of the present disclosure may further comprise at least one excipient selected from a non-sugar alcohol diluent, a disintegrant, a binder and a lubricant, preferably the non-sugar alcohol diluent is microcrystalline cellulose.

In a preferred embodiment, the compressed composition of the present disclosure may comprise from 0.1% to 15% (wt_{surfactant}/wtₜₒₜₐₗ) of surfactant, preferably from 0.5 % to 10 % (wt_{surfactant}/wtₜₒₜₐₗ), more preferably from 0.5% to 5% (wt_{surfactant}/wtₜₒₜₐₗ); even more preferably from 0.5 to 2% (wt_{surfactant}/wtₜₒₜₐₗ) of surfactant.

In a preferred embodiment, the compressed composition of the present disclosure may comprise from 0.9% to 10% (Wt_{surfactant}/wtₜₒₜₐₗ) of surfactant, preferably from 2 % to 10 % (Wtsurfactant/Wttotal), more preferably from 2% to 5% (Wtsurfactant/Wttotal).

In a preferred embodiment, the compressed composition of the present disclosure may comprise:
maltitol as sugar alcohol diluent and sodium dodecyl sulfate as surfactant; or
maltitol as sugar alcohol diluent and polysorbate 80 as surfactant;
xylitol as sugar alcohol diluent and sodium dodecyl sulfate as surfactant; or
xylitol as sugar alcohol diluent and polysorbate 80 as surfactant;
mannitol as sugar alcohol diluent and sodium dodecyl sulfate as surfactant; or
mannitol as sugar alcohol diluent and polysorbate 80 as surfactant.

In a preferred embodiment, the compressed composition of the present disclosure may comprise from 15% to 85% (wt_{sugar alcohol diluent}/wtₜₒₜₐₗ) of sugar alcohol diluent, preferably from 25% to 75% (wt_{sugar alcohol diluent}/wtₜₒₜₐₗ), more preferably from 35% to 65% (wt_{sugaralcohol diluent}/wtₜₒₜₐₗ).

In a preferred embodiment, the compressed composition of the present disclosure may comprise from 1% to 45% (wt_{macitentan}/wtₜₒₜₐₗ) of macitentan, preferably from 5% to 35% (wt_{macitentan}/wtₜₒₜₐₗ), more preferably from 5 % to 25 % (wt_{macitentan}/wtₜₒₜₐₗ).

In a preferred embodiment, the compressed composition of the present disclosure may comprise from 5% to 65% (wt_{non-sugar alcohol diluent}/wtₜₒₜₐₗ) of non-sugar alcohol diluent, preferably from 10% to 50% (wt_{non-sugar alcohol diluent}/wtₜₒₜₐₗ), more preferably from 15% to 35% (wt_{non-sugar alcohol diluent}/wtₜₒₜₐₗ).

In one embodiment, the compressed composition comprises 5% to 25% (wt_{macitentan}/wtₜₒₜₐₗ), of macitentan, 35% to 65% (wt_{sugar alcohol diluent}/wtₜₒₜₐₗ), of sugar _{alcohol} diluent and 0.5% to 5% (wt_{surfactan}t/wtₜₒₜₐₗ), of surfactant.

In one embodiment, the compressed composition further comprises at least one excipient selected from a non-sugar alcohol diluent, a disintegrant, a binder and a lubricant.

In one embodiment the non-sugar alcohol diluent is microcrystalline cellulose, the disintegrant is crospovidone, the binder is povidone and the lubricant is magnesium stearate.

In one embodiment the non-sugar alcohol diluent is microcrystalline cellulose, the disintegrant is sodium starch glycolate, the binder is povidone and the lubricant is magnesium stearate.

One aspect of the present disclosure relates to compressed compositions prepared by wet granulation.

One aspect of the present disclosure relates to a compressed composition for use in the treatment of pulmonary arterial hypertension.

One aspect of the present disclosure relates to solid oral pharmaceutical compositions comprising compressed macitentan compositions.

One aspect of the present disclosure relates to solid oral pharmaceutical compositions comprising a compressed macitentan composition for use in the treatment of pulmonary arterial hypertension.

Another aspect of the present disclosure relates to a process for preparing a compressed composition comprising:
mixing macitentan, a sugar alcohol diluent, and optionally one or more excipients selected from a non-sugar alcohol diluent, a disintegrant, a binder, a glidant;
granulating the mixture obtained in the previous step with an aqueous solution of a surfactant wherein the surfactant is sodium dodecyl sulfate, or polysorbate 80, or combinations thereof;
lubricating the granulate obtained in the previous step with magnesium stearate; compressing the lubricated granulate obtained in the previous step.

Another aspect of the present disclosure relates to a solid oral pharmaceutical composition comprising a compressed composition prepared according to a process comprising:
mixing macitentan, a sugar alcohol diluent, and optionally one or more excipients selected from a non-sugar alcohol diluent, a disintegrant, a binder, a glidant;
granulating the mixture obtained in the previous step with an aqueous solution of a surfactant wherein the surfactant is sodium dodecyl sulfate, or polysorbate 80, or combinations thereof;
lubricating the granulate obtained in the previous step with magnesium stearate;
compressing the lubricated granulate obtained in the previous step.

Another aspect of the present disclosure relates to a solid oral pharmaceutical composition for use in the treatment of pulmonary arterial hypertension comprising a compressed composition prepared according to a process comprising:
mixing macitentan, a sugar alcohol diluent, and optionally one or more excipients selected from a non-sugar alcohol diluent, a disintegrant, a binder, a glidant;
granulating the mixture obtained in the previous step with an aqueous solution of a surfactant wherein the surfactant is sodium dodecyl sulfate, or polysorbate 80, or combinations thereof;
lubricating the granulate obtained in the previous step, preferably with magnesium stearate;
compressing the lubricated granulate obtained in the previous step.

In an embodiment, the process for preparing a compressed composition, or a solid oral pharmaceutical composition for use in the treatment of pulmonary arterial hypertension wherein the sugar alcohol diluent is maltitol, xylitol, or mannitol.

In an embodiment, the process for preparing a compressed composition a solid oral pharmaceutical composition for use in the treatment of pulmonary arterial hypertension wherein the non-sugar alcohol diluent is microcrystalline cellulose.

In an embodiment, the process for preparing a compressed composition a solid oral pharmaceutical composition for use in the treatment of pulmonary arterial hypertension wherein the disintegrant is crospovidone or sodium starch glycolate or mixture thereof.

The process for preparing a compressed composition a solid oral pharmaceutical composition for use in the treatment of pulmonary arterial hypertension wherein the binder is povidone, copovidone, or mixture thereof.

In an embodiment, the non-sugar alcohol diluent refers to microcrystalline cellulose.

In an embodiment, the disintegrant refers to sodium starch glycolate, or crospovidone, or mixture thereof.

In an embodiment, the binder refers to povidone, copovidone, or mixture thereof. Preferably, the binder is povidone.

In an embodiment, the lubricant refers to calcium stearate, stearic acid, sodium stearyl fumarate or magnesium stearate, or mixture thereof. Preferably, the lubricant is magnesium stearate.

In an embodiment, the glidant refers to colloidal anhydrous silica or talc. Preferably, the glidant is anhydrous colloidal silica.

In an embodiment, which is not comprised by the claims, the compressed compositions have been found to display improved stability and fast dissolution profile as compared to compressed compositions containing 10 mg of macitentan, lactose monohydrate, microcrystalline cellulose, sodium starch glycolate, povidone, magnesium stearate and polysorbate 80.

In the context of the present disclosure a compressed composition comprises any composition comprising macitentan and pharmaceutical excipients such as tablet cores, microtablets, caplets, beads, granulates and the like. Preferably, the compressed compositions are tablet cores.

In an embodiment, the solid oral pharmaceutical composition comprising a compressed macitentan composition is in the form of any solid oral composition intended for the pharmaceutical delivery of a pharmaceutical agent, preferably as tablets, microtablets, caplets, or as beads contained in capsules, or as granulates in sachets intended to provide individual dosage forms. Most preferably, the solid oral pharmaceutical compositions are in the form of tablets.

### Brief Description of the Drawings

The following figures provide preferred embodiments for illustrating the description and should not be seen as limiting the scope of invention.

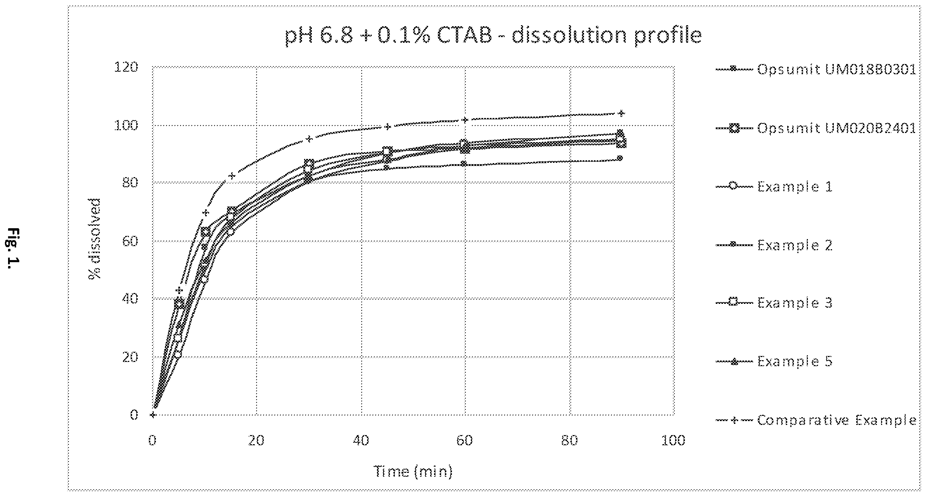

Figure 1 illustrates the dissolution profile of the disclosed compositions (Examples 1, 2, 3 and 5) as compared to the dissolution profile of the reference product Opsumit^{®}.

### Detailed Description

The present invention relates to compressed compositions comprising a therapeutically effective amount of macitentan, 15% to 85% (wt_{sugar alcohol diluent}/ wtₜₒₜₐₗ) of a sugar alcohol diluent selected from a list consisting of: maltitol, xylitol, mannitol; 0.1% to1 5% (wt_{surfactan}/wtₜₒₜₐₗ) of a surfactant selected from a list consisting of: sodium dodecyl sulfate, polysorbate 80.

It also relates to the process of obtaining said compressed compositions, oral pharmaceutical compositions comprising them, and their use in the treatment of pulmonary arterial hypertension.

The present disclosure relates to solid oral pharmaceutical compositions comprising a therapeutically effective amount of macitentan that simultaneously display improved stability properties and adequate dissolution profiles.

In an embodiment, a compressed macitentan composition (Example 1, see in table 1 the composition detail) is prepared according to the following process:
a) Macitentan, xylitol, povidone, microcrystalline cellulose, silica colloidal anhydrous, and sodium starch glycolate powders are mixed in appropriate equipment.
b) The mixture is granulated with a granulation solution prepared with SDS and water.
c) The granulate is dried at 40 °C and calibrated with a sieve aperture of a 1 mm.
d) The calibrated granulate is lubricated with magnesium stearate and thereafter compressed.

**Table 1: Compressed macitentan composition of example1**

| **Component** | **Amount (mg)** | **%** |
|---|---|---|
| Macitentan | 10.12 | 14.45 |
| Xylitol | 37.00 | 52.86 |
| Microcrystalline cellulose | 15.88 | 22.69 |
| Sodium starch glycolate | 2.10 | 3.00 |
| Povidone | 2.10 | 3.00 |
| SDS | 1.40 | 2.00 |
| Silica colloidal anhydrous | 0.70 | 1.00 |
| Magnesium stearate | 0.70 | 1.00 |
| Total | 70 | 100.00 |

In an embodiment, a compressed macitentan composition (Example 2, see in table 2 the composition detail) is prepared analogously to the composition of Example 1 by substituting xylitol for maltitol.

**Table 2: Compressed macitentan composition of example 2**

| **Component** | **Amount (mg)** | **%** |
|---|---|---|
| Macitentan | 10.12 | 14.45 |
| Maltitol | 37.00 | 52.86 |
| Microcrystalline cellulose | 15.88 | 22.69 |
| Sodium starch glycolate | 2.10 | 3.00 |
| Povidone | 2.10 | 3.00 |
| SDS | 1.40 | 2.00 |
| Silica colloidal anhydrous | 0.70 | 1.00 |
| Magnesium stearate | 0.70 | 1.00 |
| Total | 70 | 100.00 |

In an embodiment, a compressed macitentan composition (Example 3, see in table 3 the composition detail) is prepared according to the following process:
a) Macitentan, xylitol, povidone, microcrystalline cellulose, and sodium starch glycolate powders are mixed in appropriate equipment.
b) The mixture is granulated with a granulation solution prepared with polysorbate 80 and water.
c) The granulate is dried at 40 ºC and calibrated with a sieve aperture of a 1 mm.
d) The calibrated granulate is lubricated with magnesium stearate and thereafter compressed.

**Table 3: Compressed macitentan composition of example 3**

| **Component** | **Amount (mg)** | **%** |
|---|---|---|
| Macitentan | 10.12 | 14.45 |
| Xylitol | 37.00 | 52.86 |
| Microcrystalline cellulose | 16.58 | 23.69 |
| Sodium starch glycolate | 2.10 | 3.00 |
| Povidone | 2.10 | 3.00 |
| Polysorbate 80 | 1.40 | 2.00 |
| Magnesium stearate | 0.70 | 1.00 |
| Total | 70 | 100.00 |

In an embodiment, a compressed macitentan composition (Example 4, see in table 4 the composition detail) is prepared analogously to the composition of Example 3 by substituting xylitol for maltitol.

**Table 4: Compressed macitentan composition of example 4**

| **Component** | **Amount (mg)** | **%** |
|---|---|---|
| Macitentan | 10.12 | 14.45 |
| Maltitol | 37.00 | 52.86 |
| Microcrystalline cellulose | 16.58 | 23.69 |
| Sodium starch glycolate | 2.10 | 3.00 |
| Povidone | 2.10 | 3.00 |
| Polysorbate 80 | 1.40 | 2.00 |
| Magnesium stearate | 0.70 | 1.00 |
| Total | 70 | 100.00 |

In an embodiment, a compressed macitentan composition (Example 5, see in table 5 the composition detail) is prepared analogously to composition of Example 1 by substituting xylitol for mannitol.

**Table 5: Compressed macitentan composition of example 5**

| **Component** | **Amount (mg)** | **%** |
|---|---|---|
| Macitentan | 10.12 | 14.45 |
| Mannitol | 37.00 | 52.86 |
| Microcrystalline cellulose | 15.88 | 22.69 |
| Sodium starch glycolate | 2.10 | 3.00 |
| Povidone | 2.10 | 3.00 |
| SDS | 1.40 | 2.00 |
| Silica colloidal anhydrous | 0.70 | 1.00 |
| Magnesium stearate | 0.70 | 1.00 |
| Total | 70 | 100.00 |

In an embodiment, a compressed macitentan composition (Example 6, see in table6 the composition detail) is prepared analogously to composition of Example 3.

**Table 6: Compressed macitentan composition of example 6**

| **Component** | **Amount (mg)** | **%** |
|---|---|---|
| Macitentan | 10.00 | 14.29 |
| Mannitol | 37.00 | 52.86 |
| Microcrystalline cellulose | 16.42 | 23.46 |
| Crospovidone | 3.50 | 5.00 |
| Povidone | 1.75 | 2.50 |
| Tween 80 | 0.63 | 0.90 |
| Magnesium stearate | 0.70 | 1.00 |
| Total | 70 | 100.00 |

In an embodiment, a compressed macitentan composition (Example 7, see in table 7 the composition detail) is prepared analogously to composition of Example 3.

**Table 7: Compressed macitentan composition of example 7**

| **Component** | **Amount (mg)** | **%** |
|---|---|---|
| Macitentan | 10.12 | 14.45 |
| Mannitol | 37.00 | 52.86 |
| Microcrystalline cellulose | 16.58 | 23.69 |
| Sodium starch glycolate | 2.10 | 3.00 |
| Povidone | 2.10 | 3.00 |
| Tween 80 | 1.40 | 2.00 |
| Magnesium stearate | 0.70 | 1.00 |
| Total | 70 | 100.00 |

In an embodiment, a compressed macitentan composition (Example 8, see in table 8 the composition detail) is prepared analogously to composition of Example 3.

**Table 8: Compressed macitentan composition of example 8**

| **Component** | **Amount (mg)** | **%** |
|---|---|---|
| Macitentan | 10.12 | 14.45 |
| Mannitol | 37.00 | 52.86 |
| Microcrystalline cellulose | 17.28 | 24.69 |
| Crospovidone | 2.10 | 3.00 |
| Povidone | 2.10 | 3.00 |
| Tween 80 | 0.70 | 1.00 |
| Magnesium stearate | 0.70 | 1.00 |
| Total | 70 | 100.00 |

A comparative compressed macitentan composition (see in table 9 the composition detail) was prepared according to the following process:
a) Macitentan, lactose monohydrate, povidone, microcrystalline cellulose, and sodium starch glycolate powders were mixed in appropriate equipment.
b) The mixture was granulated with a granulation solution prepared with polysorbate 80 and water. The granulate was dried at 40 ºC and calibrated with a sieve aperture of a 1 mm.
c) The calibrated granulate was lubricated with magnesium stearate and compressed.

**Table 9: Compressed macitentan composition (Comparative example)**

| **Component** | **Amount (mg)** | **%** |
|---|---|---|
| Macitentan | 10.12 | 14.45 |
| Lactose monohydrate | 37.00 | 52.86 |
| Microcrystalline cellulose | 16.58 | 23.69 |
| Sodium starch glycolate | 2.10 | 3.00 |
| Povidone | 2.10 | 3.00 |
| Polysorbate 80 | 1.40 | 2.00 |
| Magnesium stearate | 0.70 | 1.00 |
| Total | 70 | 100.00 |

In an embodiment, the stability of the compositions was analysed. The compositions described in examples 1 to 4 and 7 and the composition of the comparative example were submitted to stress conditions in order to study the product behaviour and degradation profile throughout the time. The test batches were stored in closed bottles and left under two different conditions (a) 40°C with 75% relative humidity, (b) 85% relative humidity. Each test batch was analysed after 1.5 months of exposition and/or after 3 months of exposition.

In an embodiment, each composition was analysed using a High Performance Liquid Chromatography system with a UV detector at 260 nm. For the samples analysis was used a validated and stability indicating methodology, and the quantification was performed using a characterized working standard. The MCT-IMA impurity, which is the prevalent impurity of Macitentan, and the total of impurities were quantified.

**Table 10: Data related to the stability of the composition of the example 1-4 and 7, with SDS or polysorbate and maltitol, xylitol, mannitol, and the comparative example (detailed on table 9) at 75% RH and 40 °C**

| 40°C/75%RH Closed Bottle | Composition Ex. 1 | | Composition Ex. 2 | | Composition Ex. 3 | | Composition Ex. 4 | | Composition Ex. 7 | | Comparative Example | |
|---|---|---|---|---|---|---|---|---|---|---|---|---|
| Time (months) | 1.5 | 3 | 1.5 | 3 | 1.5 | 3 | 1.5 | 3 | 1.5 | 3 | 1.5 | 3 |
| MCT-IMA impurity (%) | 0.057 | 0.081 | 0.051 | 0.064 | 0.065 | 0.095 | 0.065 | 0.103 | 0.072 | - | 0.086 | 0.119 |
| Total Impurities (%) | 0.103 | 0.122 | - | 0.144 | 0.106 | 0.139 | 0.100 | 0.147 | 0.123 | - | 0.134 | 0.214 |

**Table 11: Data related to the stability of the composition of the example 1-4 and 7, with SDS or polysorbate and maltitol, xylitol, mannitol, and the comparative example (detailed on table 9) at 85% RH**

| 85% RH | Composition of Example 1 | Composition of Example 2 | Composition of Example 3 | Composition of Example 4 | Composition of Example 7 | Comparative Example |
|---|---|---|---|---|---|---|
| Time (months) | 3 | 3 | 3 | 3 | 3 | 3 |
| Total Impurities (%) | 0.089 | 0.080 | 0.045 | 0.045 | 0.046 | 0.100 |

In an embodiment, the compressed macitentan compositions of examples 1 to 4 and 7 exhibit enhanced stability as compared to the composition of the comparative example (detailed on table 9). Table 10 shows that after 3 months under 40°C with 75% relative humidity, compositions of examples 1 to 4 and 7 exhibit lower MCT-IMA impurity level and lower total impurities level as compared to the composition of the comparative example. Table 11 shows that after 3 months under 85% relative humidity, compositions of examples 1 to 4 and 7 exhibit lower total impurities level than the composition of the comparative example.

In an embodiment, the stability of the compositions was analysed. The compositions described in examples 1, 2 and 5 and the composition of the comparative example were submitted to stress conditions in order to study the product behaviour and degradation profile throughout the time. The test batches were stored in closed bottles and left under 60°C. Each test batch was analysed twice, once after 1.5 months of exposition and once after 3 months of exposition.

In an embodiment, each composition was analysed using a High-Performance Liquid Chromatography system with a UV detector at 260 nm. For the samples analysis was used a validated and stability indicating methodology, and the quantification was performed using a characterized working standard. The MCT-IMA impurity, which is the prevalent impurity of Macitentan and the total of impurities were quantified.

**Table 12: Data related to the stability of the composition of the example 1, 2, 5, with SDS and xylitol, maltitol or mannitol, and the comparative example at 60°C**

| 60°C | Composition 1 | | Composition 2 | | Composition 5 | | Comparative Example | |
|---|---|---|---|---|---|---|---|---|
| Time (months) | 1.5 | 3 | 1.5 | 3 | 1.5 | 3 | 1.5 | 3 |
| MCT-IMA impurity (%) | 0.402 | 0.601 | 0.114 | 0.426 | 1.184 | 1.733 | 2.867 | 5.752 |
| Total Impurities (%) | 0.529 | 0.858 | 0.336 | 0.470 | 1.428 | 2.376 | 3.074 | 6.406 |

Table 12 shows that at 60°C, the compressed macitentan compositions of Examples 1, 2 and 5 exhibit enhanced stability as compared to the composition of the comparative example (detailed on table 9). After 1.5 months and 3 months under 60°C, compositions of examples 1, 2 and 5 exhibit lower level of MCT-IMA impurity and lower level of total impurities than the composition of the comparative example.

**Table 13: Data related to the stability of the composition of the example 6, at 40 °C and 30 °C**

| Conditions | 40 °C / 75% HR | | 30 °C / 75% HR | |
|---|---|---|---|---|
| Time (months) | 6 | 12 | 6 | 12 |
| MCT-IMA impurity (%) | <0.05% | n/a | <0.05% | <0.05% |
| Total Impurities (%) | <0.05% | n/a | <0.05% | <0.05% |

Table 13 shows that at 40°C or 30°C, the compressed macitentan compositions of Examples 6 exhibit enhanced stability as compared to the composition of the comparative example (detailed on table 9).

A dissolution test was also performed. In the dissolution test of the disclosed examples were performed in 900 mL of dissolution medium at 37 °C ± 0.5ºC, using USP Apparatus 2 (paddles) method at a rotation speed of 50 rpm. Samples are removed after 5, 10, 20, 30, 45, 60 and 90 minutes from test initiation and analyzed for dissolved macitentan using a suitable HPLC method at 260 nm. pH 6.8 phosphate buffer + 0.1% CTAB had been used as dissolution medium, with adequate discrimination ability. The reported dissolution results are average values from six tablets.

Table 14 shows the dissolution profiles of the example compositions as compared with the reference drug product (Opsumit^{®}). The dissolution tests were performed under same conditions, using a similarity factor (f2). The similarity factor (f2) is a measurement of the similarity in percentage of dissolution between two curves. Two dissolution profiles are considered similar when the f2 value is ≥ 50.

**Table 14: Data related to the dissolution profiles of an oral solid composition of the example 1, 2, 3 ,5 and the composition of the Opsumit^{®} tablets**

| *ƒ2*(%) | Example 1 | Example 2 | Example 3 | Example 5 |
|---|---|---|---|---|
| Opsumit^{®} | 59 | 66 | 77 | 73 |

The compressed macitentan compositions tested showed good similarity when compared to commercial solid oral compositions comprising macitentan (Opsumit^{®} tablets).

**Table 15: Data related to the dissolution profiles of an oral solid composition of the example 6 in 0.1N HCl**

| **Time (min)** | **%** |
|---|---|
| 0 | 0 |
| 5 | 48 |
| 10 | 67 |
| 15 | 74 |
| 30 | 84 |
| 45 | 88 |
| 60 | 90 |
| 90 | 92 |

**Table 16: Data related to the dissolution profiles of an oral solid composition of the example 6 in phosphate buffer, pH 6.8, 0.02% CTAB and 50 rpm**

| Time (min) | T0 | (30 °C/75%HR) after 6 months storage | (40 °C /75%HR) after 6 months storage |
|---|---|---|---|
| 0 | 0 | 0 | 0 |
| 5 | 42 | 40 | 38 |
| 10 | 60 | 58 | 61 |
| 15 | 67 | 66 | 71 |
| 30 | 79 | 77 | 85 |
| 45 | 85 | 82 | 91 |
| 60 | 89 | 86 | 95 |
| 90 | 93 | 90 | 99 |

**Table 17: Data related to the dissolution profiles of an oral solid composition of the example 6, 7 and 8 in phosphate buffer pH 6.8, 0.1% CTAB and 50 rpm**

| Time (min) | Example 6 | Example 7 | Example 8 |
|---|---|---|---|
| | T0 | T0 | T0 |
| 0 | 0 | 0 | 0 |
| 5 | 35 | 22 | 24 |
| 10 | 51 | 50 | 50 |
| 15 | 60 | 65 | 62 |
| 30 | 74 | 83 | 77 |
| 45 | 81 | 92 | 85 |
| 60 | 84 | 98 | 89 |
| 90 | 91 | 100 | 94 |

**Table 18: Data related to the dissolution profiles of an oral solid composition of the example 6, 7 and 8 and the composition of the Opsumit^{®} tablets**

| *f2*(%) | Example 6 | Example 7 | Example 8 |
|---|---|---|---|
| Opsumit^{®} | 60 | 68 | 62 |

Where ranges are given, endpoints are included. Furthermore, it is to be understood that unless otherwise indicated or otherwise evident from the context and/or the understanding of one of ordinary skill in the art, values that are expressed as ranges can assume any specific value within the stated ranges in different embodiments of the invention, to the tenth of the unit of the lower limit of the range, unless the context clearly dictates otherwise. It is also to be understood that unless otherwise indicated or otherwise evident from the context and/or the understanding of one of ordinary skill in the art, values expressed as ranges can assume any subrange within the given range, wherein the endpoints of the subrange are expressed to the same degree of accuracy as the tenth of the unit of the lower limit of the range.

The term "comprising" whenever used in this document is intended to indicate the presence of stated features, integers, steps, components, but not to preclude the presence or addition of one or more other features, integers, steps, components or groups thereof.

## Claims

1. A compressed composition comprising a therapeutically effective amount of macitentan;
15% to 85% (wt_{sugar alcohol diluent}/wtₜₒₜₐₗ) of a sugar alcohol diluent selected from a list consisting of: maltitol, xylitol, mannitol;
0.1% to 15% (wt_{surfactant}/wtₜₒₜₐₗ) of a surfactant selected from a list consisting of: sodium dodecyl sulfate, polysorbate 80.

2. The compressed composition according to previous claim comprising 0.5% to 15% (wt_{surfactant}/wtₜₒₜₐₗ) of surfactant.

3. The compressed composition according to any of the previous claims comprising from 0.9% to 10% (wt_{surfactant}/wtₜₒₜₐₗ) of surfactant, preferably from 2 % to 10 % (Wtsurfactant/Wttotal), more preferably from 2% to 5% (Wtsurfactant/Wttotal).

4. The compressed composition according to any of the previous claims comprising from 0.5 to 2% (wt_{surfactant}/wtₜₒₜₐₗ) of surfactant.

5. The compressed composition according to the previous claims comprising
maltitol as sugar alcohol diluent and sodium dodecyl sulfate as surfactant; or
maltitol as sugar alcohol diluent and polysorbate 80 as surfactant;
xylitol as sugar alcohol diluent and sodium dodecyl sulfate as surfactant; or
xylitol as sugar alcohol diluent and polysorbate 80 as surfactant;
mannitol as sugar alcohol diluent and sodium dodecyl sulfate as surfactant; or
mannitol as sugar alcohol diluent and polysorbate 80 as surfactant.

6. The compressed composition according to any of the previous claims wherein the compressed composition comprises from 5% to 65% (wt_{non-sugar alcohol diluent}/wtₜₒₜₐₗ) of non-sugar alcohol diluent, preferably from 10% to 50% (wt_{non-sugar alcohol diluent}/wtₜₒₜₐₗ), more preferably from 15% to 35% (wt_{non-sugar alcohol diluent}/wtₜₒₜₐₗ).

7. The compressed composition according to any of the previous claims further comprising microcrystalline cellulose as a non-sugar alcohol diluent.

8. The compressed composition according to any of the previous claims comprising from 25% to 75% (wt_{sugar alcohol diluent}/wtₜₒₜₐₗ) of the sugar alcohol diluent, more preferably from 35% to 65% (wt_{sugar alcohol diluent}/wtₜₒₜₐₗ).

9. The compressed composition according to any of the previous claims comprising from 1% to 45% (wt_{macitentan}/wtₜₒₜₐₗ) of macitentan, preferably from 5% to 35% (Wtmacitentan/Wttotal), more preferably from 5 % to 25 % (Wtmacitentan/Wttotal).

10. The compressed composition according to any of the previous claims wherein the compressed composition comprises from 5% to 25% (wt_{macitentan}/wtₜₒₜₐₗ) of macitentan, from 35% to 65% (wt_{sugar alcohol diluent}/wtₜₒₜₐₗ) of sugar alcohol diluent, and from 0.5% to 5% (wt_{surfactant}/wtₜₒₜₐₗ) of surfactant.

11. The compressed composition according to any of the previous claims further comprising at least one excipient selected from a disintegrant, a binder or a lubricant.

12. The compressed composition according to any of the previous claims wherein the non-sugar alcohol diluent is microcrystalline cellulose, the disintegrant is crospovidone or sodium starch glycolate, the binder is povidone, and the lubricant is magnesium stearate.

13. The compressed composition according to any of the previous claims wherein the composition is prepared by wet granulation.

14. The compressed composition according to any of the previous claims for use in the treatment of pulmonary arterial hypertension.

15. A process for preparing a compressed composition, or a solid oral pharmaceutical composition, or a solid oral pharmaceutical composition for use in the treatment of pulmonary arterial hypertension, comprising:
mixing macitentan, a sugar alcohol diluent, and optionally one or more excipients selected from a non-sugar alcohol diluent, a disintegrant, a binder, a glidant;
granulating the mixture obtained in the previous step with an aqueous solution of a surfactant wherein the surfactant is sodium dodecyl sulfate, or polysorbate 80;
lubricating the granulate obtained in the previous step, preferably with magnesium stearate;
wherein the sugar alcohol diluent is selected from a list consisting of: maltitol, xylitol, mannitol;
compressing the lubricated granulate obtained in the previous step.

## Patentansprüche

1. Eine gepresste Zusammensetzung, umfassend eine therapeutisch wirksame Menge an Macitentan;
15 % bis 85 % (wt_{zuckeralkoholisches Verdünnungsmittel}/wt_{gesamt}) eines zuckeralkoholischen Verdünnungsmittels, ausgewählt aus einer Liste bestehend aus: Maltitol, Xylitol, Mannitol;
0,1 % bis 15 % (wt_{Tensid}/wt_{gesamt}) eines Tensids, ausgewählt aus einer Liste bestehend aus: Natriumdodecylsulfat, Polysorbat 80.

2. Die gepresste Zusammensetzung nach dem vorangehenden Anspruch, umfassend 0,5 % bis 15 % (wt_{Tensid}/wt_{gesamt}) Tensid.

3. Die gepresste Zusammensetzung nach einem der vorangehenden Ansprüche, umfassend von 0,9 % bis 10% (wt_{Tensid}/wt_{gesamt}) Tensid, bevorzugt von 2% bis 10% (wt_{Tensid}/wt_{gesamt}), besonders bevorzugt von 2 % bis 5 % (wt_{Tensid}/wt_{gesamt}).

4. Die gepresste Zusammensetzung nach einem der vorangehenden Ansprüche umfassend von 0,5 % bis 2 % (wt_{Tensid}/wt_{gesamt}) eines Tensids.

5. Die gepresste Zusammensetzung nach den vorangehenden Ansprüchen, umfassend
Maltitol als zuckeralkoholisches Verdünnungsmittel und Natriumdodecylsulfat als Tensid; oder
Maltitol als zuckeralkoholisches Verdünnungsmittel und Polysorbat 80 als Tensid;
Xylitol als zuckeralkoholisches Verdünnungsmittel und Natriumdodecylsulfat als Tensid; oder
Xylitol als zuckeralkoholisches Verdünnungsmittel und Polysorbat 80 als Tensid;
Mannitol als zuckeralkoholisches Verdünnungsmittel und Natriumdodecylsulfat als Tensid; oder
Mannitol als zuckeralkoholisches Verdünnungsmittel und Polysorbat 80 als Tensid.

6. Die gepresste Zusammensetzung nach einem der vorangehenden Ansprüche, wobei die gepresste Zusammensetzung von 5% bis 65 % (wt_{nicht zuckeralkoholisches Verdünnungsmittel}/wt_{gesamt}) nicht zuckeralkoholisches Verdünnungsmittel, bevorzugt von 10% bis 50 % (wt_{nicht zuckeralkoholisches Verdünnungsmittel}/wt_{gesamt}), besonders bevorzugt von 15 % bis 35 % (wt_{nicht zuckeralkoholisches Verdünnungsmittel}/wt_{gesamt}) umfasst.

7. Die gepresste Zusammensetzung nach einem der vorangehenden Ansprüche, ferner umfassend mikrokristalline Cellulose als nicht zuckeralkoholisches Verdünnungsmittel.

8. Die gepresste Zusammensetzung nach einem der vorangehenden Ansprüche, umfassend von 25 % bis 75 % (wt_{zuckeralkoholisches Verdünnungsmittel}/wt_{gesamt}) zuckeralkoholisches Verdünnungsmittel, besonders bevorzugt von 35 % bis 65% (wt_{zuckeralkoholisches Verdünnungsmittel}/wt_{gesamt}).

9. Die gepresste Zusammensetzung nach einem der vorangehenden Ansprüche, umfassend von 1 % bis 45 % (wt_{Macitentan}/wt_{gesamt}) Macitentan, bevorzugt von 5 % bis 35 % (wt_{Macitentan}/wt_{gesamt}), besonders bevorzugt von 5 % bis 25 % (wt_{Macitentan}/wt_{gesamt}).

10. Die gepresste Zusammensetzung nach einem der vorangehenden Ansprüche, wobei die gepresste Zusammensetzung von 5% bis 25% (wt_{Macitentan}/wt_{gesamt}) Macitentan, von 35 % bis 65 % (wt_{zuckeralkoholisches Verdünnungsmittel}/wt_{gesamt}) _{zuckeralkoholisches} Verdünnungsmittel, und von 0,5 % bis 5 % (wt_{Tensid}/wt_{gesamt}) Tensid umfasst.

11. Die gepresste Zusammensetzung nach einem der vorangehenden Ansprüche, ferner umfassend mindestens einen Hilfsstoff, ausgewählt aus einem Sprengmittel, einem Bindemittel oder einem Gleitmittel.

12. Die gepresste Zusammensetzung nach einem der vorangehenden Ansprüche, wobei das nicht zuckeralkoholische Verdünnungsmittel mikrokristalline Cellulose ist, das Sprengmittel Crospovidon oder Natriumstärkeglykolat ist, das Bindemittel Povidon ist und das Gleitmittel Magnesiumstearat ist.

13. Die gepresste Zusammensetzung nach einem der vorangehenden Ansprüche, wobei die Zusammensetzung durch Nassgranulierung hergestellt wird.

14. Die gepresste Zusammensetzung nach einem der vorangehenden Ansprüche zur Verwendung bei der Behandlung von pulmonal arterieller Hypertonie.

15. Ein Verfahren zur Herstellung einer gepressten Zusammensetzung, oder einer festen oralen pharmazeutischen Zusammensetzung, oder einer festen oralen pharmazeutischen Zusammensetzung zur Verwendung bei der Behandlung von pulmonal arterieller Hypertonie, umfassend:
Mischen von Macitentan, einem zuckeralkoholischen Verdünnungsmittel, und gegebenenfalls einem oder mehreren Hilfsstoffen, ausgewählt aus einem nicht zuckeralkoholischen Verdünnungsmittel, einem Sprengstoff, einem Bindemittel, einem Gleitmittel;
Granulieren der im vorherigen Schritt erhaltenen Mischung mit einer wässrigen Lösung eines Tensids, wobei das Tensid Natriumdodecylsulfat oder Polysorbat 80 ist;
Aufbringen des Gleitmittels auf das im vorherigen Schritt erhaltene Granulat, bevorzug mit Magnesiumstearat;
wobei das zuckeralkoholische Verdünnungsmittel aus einer Liste ausgewählt wird, bestehend aus: Maltitol, Xylitol, Mannitol;
Pressen des im vorherigen Schritt erhaltenen geschmierten Granulats.

## Revendications

1. Une composition comprimée comprenant une quantité thérapeutiquement efficace de macitentan ;
15% à 85% (poids_{diluant d'alcool de sucre}/poidsₜₒₜₐₗ) d'un diluant d'alcool de sucre choisi parmi une liste consistant en : maltitol, xylitol, mannitol ;
0,1% à 15% (poids_{tensioactif}/poidsₜₒₜₐₗ) d'un tensioactif choisi parmi une liste consistant en : dodécylsulfate de sodium, polysorbate 80.

2. La composition comprimée selon la revendication précédente comprenant 0,5% à 15% (poids_{tensioactif}/poidsₜₒₜₐₗ) de tensioactif.

3. La composition comprimée selon l'une quelconque des revendications précédentes comprenant de 0,9% à 10% (poids_{tensioactif}/poidsₜₒₜₐₗ) de tensioactif, préférablement de 2% à 10% (poids_{tensioactif}/poidsₜₒₜₐₗ), plus préférablement de 2% à 5% (poids_{tensioactif}/poidsₜₒₜₐₗ).

4. La composition comprimée selon l'une quelconque des revendications précédentes comprenant de 0,5% à 2% (poids_{tensioactif}/poidsₜₒₜₐₗ) de tensioactif.

5. La composition comprimée selon les revendications précédentes comprenant
du maltitol en tant que diluant d'alcool de sucre et du dodécylsulfate de sodium en tant que tensioactif ; ou
du maltitol en tant que diluant d'alcool de sucre et du polysorbate 80 en tant que tensioactif ;
du xylitol en tant que diluant d'alcool de sucre et du dodécylsulfate de sodium en tant que tensioactif ; ou
du xylitol en tant que diluant d'alcool de sucre et du polysorbate 80 en tant que tensioactif ;
du mannitol en tant que diluant d'alcool de sucre et du dodécylsulfate de sodium en tant que tensioactif ; ou
du mannitol en tant que diluant d'alcool de sucre et du polysorbate 80 en tant que tensioactif.

6. La composition comprimée selon l'une quelconque des revendications précédentes dans laquelle la composition comprimée comprend de 5% à 65% (poids_{diluant d'alcool sans sucre}/poidsₜₒₜₐₗ) de diluant d'alcool sans sucre, préférablement de 10% à 50% (poids_{diluant d'alcool sans sucre}/poidsₜₒₜₐₗ), plus préférablement de 15% à 35% (poids_{diluant d'alcool sans sucre}/poidsₜₒₜₐₗ).

7. La composition comprimée selon l'une quelconque des revendications précédentes comprenant également de la cellulose microcristalline en tant que diluant d'alcool sans sucre.

8. La composition comprimée selon l'une quelconque des revendications précédentes comprenant de 25% à 75% (poids_{diluant d'alcool de sucre}/poidsₜₒₜₐₗ) de diluant d'alcool de sucre, plus préférablement de 35% à 65% (poids_{diluant d'alcool desucre}/poidsₜₒₜₐₗ).

9. La composition comprimée selon l'une quelconque des revendications précédentes comprenant de 1% à 45% (poids_{macitentan}/poidsₜₒₜₐₗ) de macitentan, préférablement de 5% à 35% (poids_{racitentan}/poidsₜₒₜₐₗ), plus préférablement de 5% à 25% (poids_{macitentan}/poidsₜₒₜₐₗ).

10. La composition comprimée selon l'une quelconque des revendications précédentes dans laquelle la composition comprimée comprend de 5% à 25% (poids_{macitentan}/poidsₜₒₜₐₗ) de macitentan, de 35% à 65% (poids_{diluant d'alcool de sucre}/poidsₜₒₜₐₗ) de diluant d'alcool de sucre, et de 0,5% à 5% (poids_{tensioactif}/poidsₜₒₜₐₗ) de tensioactif.

11. La composition comprimée selon l'une quelconque des revendications précédentes comprenant également au moins un excipient choisi parmi un désintégrant, un liant ou un lubrifiant.

12. La composition comprimée selon l'une quelconque des revendications précédentes dans laquelle le diluant d'alcool sans sucre est de la cellulose microcristalline, le désintégrant est de la crospovidone ou du glycolate d'amidon sodique, le liant est de la povidone, et le lubrifiant est du stéarate de magnésium.

13. La composition comprimée selon l'une quelconque des revendications précédentes dans laquelle la composition est préparée par granulation humide.

14. La composition comprimée selon l'une quelconque des revendications précédentes destinée à être utilisée pour le traitement de l'hypertension artérielle pulmonaire.

15. Un processus de préparation d'une composition comprimée, ou d'une composition pharmaceutique orale solide, ou d'une composition pharmaceutique orale solide destinée à être utilisée pour le traitement de l'hypertension artérielle pulmonaire, comprenant :
mélanger le macitentan, un diluant d'alcool de sucre, et facultativement un ou plusieurs excipients choisis parmi un diluant d'alcool sans sucre, un désintégrant, un liant, un agent de glissement ;
granuler le mélange obtenu lors de l'étape précédente avec une solution aqueuse d'un tensioactif dans lequel le tensioactif est du dodécylsulfate de sodium, ou du polysorbate 80 ;
lubrifier le granulé obtenu lors de l'étape précédente, préférablement avec du stéarate de magnésium ;
dans lequel le diluant d'alcool de sucre est choisi parmi une liste consistant en : maltitol, xylitol, mannitol ;
comprimer le granulé lubrifié obtenu lors de l'étape précédente.
